# EUROPEAN PATENT SPECIFICATION

(11) **EP 2 007 910 B1**
(45) Date of publication and mention of the grant of the patent: **02.11.2016**
(21) Application number: 07730669.4
(22) Date of filing: 02.04.2007
(51) Int. Cl.: C12Q 1/70

(54) **METHOD AND MICROARRAY FOR DETECTING HERPESVIRUSES**
VERFAHREN UND MIKROARRAY ZUM NACHWEIS VON HERPESVIREN
PROCÉDÉ ET MICRORÉSEAU DESTINÉS À LA DÉTECTION DE VIRUS DE L'HERPÈS

(30) Priority: 31.03.2006 US 788307 P
(43) Date of publication of application: 31.12.2008
(73) Proprietor: Mobidiag Oy, 02150 Espoo (FI)
(72) Inventor: JÄÄSKELÄINEN, Anne, J., 02610 Espoo (FI); PIIPARINEN, Heli, 04130 Sipoo (FI); LAPPALAINEN, Maija, 02180 Espoo (FI); VAHERI, Antti, 00330 Helsinki (FI)
(74) Representative: Kolster Oy Ab
(86) International application number: PCT/FI2007/050182
(87) International publication number: WO 2007/113388

(56) References cited:
- WO-A-01/77172
- WO-A-2004/016219
- WO-A2-02/10449
- WO-A2-2005/116250
- PIIPARINEN H ET AL: "GENOTYPING OF HERPES SIMPLEX VIRUSES BY POLYMERASE CHAIN REACTION" ARCHIVES OF VIROLOGY, NEW YORK, NY, US, vol. 119, no. 3-4, 1991, pages 275-283, XP009095637 ISSN: 0304-8608 cited in the application
- GIBBS J S ET AL: "SEQUENCE AND MAPPING ANALYSES OF THE HERPES SIMPLEX VIRUS DNA POLYMERASE GENE PREDICT A CARBOXYL-TERMINAL SUBSTRATE BINDING DOMAIN" PROCEEDINGS OF THE NATIONAL ACADEMY OF SCIENCES OF THE UNITED STATES OF AMERICA, vol. 82, no. 23, 1985, pages 7969-7973, XP002468552 ISSN: 0027-8424
- PASTINEN T ET AL: "A system for specific, high-throughput genotyping by allele-specific primer extension on microarrays" GENOME RESEARCH, COLD SPRING HARBOR LABORATORY PRESS, WOODBURY, NY, US, vol. 10, no. 7, July 2000 (2000-07), pages 1031-1042, XP002228091 ISSN: 1088-9051
- FOELDES-PAPP ZENO ET AL: "Detection of multiple human herpes viruses by DNA microarray technology" MOLECULAR DIAGNOSIS, NAPERVILLE, IL, US, vol. 8, no. 1, 2004, pages 1-9, XP009086578 ISSN: 1084-8592
- JAASKELAINEN ET AL: "Multiplex-PCR and oligonucleotide microarray for detection of eight different herpesviruses from clinical specimens" JOURNAL OF CLINICAL VIROLOGY, ELSEVIER, AMSTERDAM,, NL, vol. 37, no. 2, 26 July 2006 (2006-07-26), pages 83-90, XP005670891 ISSN: 1386-6532
- DATABASE EMBL [Online] 23 January 2009 'Sequence 541355 from Patent WO2005116250.' Retrieved from EBI, accession no. EM_PAT:GM590677 Database accession no. GM590677
- DATABASE GENESEQ [Online] 15 July 2002 'Mouse spliced transcript detection oligonucleotide SEQ ID NO:30691.' Retrieved from EBI, accession no. GSN:ABN57943 Database accession no. ABN57943
- DATABASE EMBL [Online] 30 January 2004 'Sequence 1926 from Patent WO0210449.' Retrieved from EBI, accession no. EM_PAT:CQ532291 Database accession no. CQ532291
- MARK J. ESPY ET AL: 'Diagnosis of Varicella-Zoster Virus Infections in the Clinical Laboratory by LightCycler PCR' JOURNAL OF CLINICAL MICROBIOLOGY, [Online] 01 January 2000, UK, pages 3187 - 3189, XP055115543 Retrieved from the Internet: <URL:http://ukpmc.ac.uk/articlerender.cgi?a rtid=246564>
- M N PRICHARD ET AL: 'A recombinant human cytomegalovirus with a large deletion in UL97 has a severe replication deficiency' JOURNAL OF VIROLOGY, [Online] 01 July 1999, UNITED STATES, pages 5663 - 5670, XP055115548 Retrieved from the Internet: <URL:http://www.pubmedcentral.nih.gov/artic lerender.fcgi?artid=112625&tool=pmcentrez&r endertype=abstract>
- R. M. WADOWSKY ET AL: 'Measurement of Epstein-Barr Virus DNA Loads in Whole Blood and Plasma by TaqMan PCR and in Peripheral Blood Lymphocytes by Competitive PCR' JOURNAL OF CLINICAL MICROBIOLOGY vol. 41, no. 11, 01 November 2003, pages 5245 - 5249, XP055037377 DOI: 10.1128/JCM.41.11.5245-5249.2003 ISSN: 0095-1137
- ZHEN ZHU ET AL: "The human herpesvirus 6 G protein-coupled receptor homolog U51 positively regulates virus replication and enhances cell-cell fusion in vitro", JOURNAL OF VIROLOGY, THE AMERICAN SOCIETY FOR MICROBIOLOGY, US, vol. 79, no. 18, 1 September 2005 (2005-09-01), pages 11914-11924, XP002460124, ISSN: 0022-538X, DOI: 10.1128/JVI.79.18.11914-11924.2005
- YOSHIKAWA TETSUSHI ET AL: "Detection of human herpesvirus 7 DNA by loop-mediated isothermal amplification", JOURNAL OF CLINICAL MICROBIOLOGY, AMERICAN SOCIETY FOR MICROBIOLOGY, US, vol. 42, no. 3, 1 March 2004 (2004-03-01), pages 1348-1352, XP002534230, ISSN: 0095-1137, DOI: 10.1128/JCM.42.3.1348-1352.2004
- X BAI ET AL: 'Quantitative polymerase chain reaction for human herpesvirus diagnosis and measurement of Epstein-Barr virus burden in posttransplant lymphoproliferative disorder' CLINICAL CHEMISTRY, [Online] 01 October 1997, UNITED STATES, pages 1843 - 1849, XP055115512 Retrieved from the Internet: <URL:http://www.clinchem.org/cgi/content/ab stract/43/10/1843>

### SEQUENCE LISTING

<110> Licentia Ltd.
<120> Method and microarray for detecting herpesviruses
<130> LIC13PCT
<150> 60/788,307
   <151> 2006-03-31
<160> 29
<170> PatentIn version 3.4
<210> 1
   <211> 21
   <212> DNA
   <213> Artificial
<220>
   <223> forward primer
<400> 1
   aaggaggcgc ccaagcgccc g 21
<210> 2
   <211> 23
   <212> DNA
   <213> Artificial
<220>
   <223> T3 RNA polymerase promoter sequence
<400> 2
   aattaaccct cactaaaggg aga 23
<210> 3
   <211> 21
   <212> DNA
   <213> Artificial
<220>
   <223> reverse primer
<400> 3
   tggggtacag gctggcaaag t 21
<210> 4
   <211> 16
   <212> DNA
   <213> Artificial
<220>
   <223> oligonucleotide
<400> 4
   gtccttgacc ccactt 16
<210> 5
   <211> 17
   <212> DNA
   <213> Artificial
<220>
   <223> oligonucleotide
<400> 5
   aggataagga cgacgac 17
<210> 6
   <211> 20
   <212> DNA
   <213> Artificial
<220>
   <223> forward primer
<400> 6
   ccattttctc gccgatttta 20
<210> 7
   <211> 20
   <212> DNA
   <213> Artificial
<220>
   <223> reverse primer
<400> 7
   gccgcatttg aacgttttat 20
<210> 8
   <211> 17
   <212> DNA
   <213> Artificial
<220>
   <223> oligonucleotide
<400> 8
   acctcgtacg ctttttg 17
<210> 9
   <211> 20
   <212> DNA
   <213> Artificial
<220>
   <223> forward primer
<400> 9
   gtacaacagc gtgtcgtgct 20
<210> 10
   <211> 20
   <212> DNA
   <213> Artificial
<220>
   <223> reverse primer
<400> 10
   caccggccat caagtttatc 20
<210> 11
   <211> 19
   <212> DNA
   <213> Artificial
<220>
   <223> oligonucleotide
<400> 11
   gtagaagttc ttcagctgc 19
<210> 12
   <211> 19
   <212> DNA
   <213> Artificial
<220>
   <223> forward primer
<400> 12
   cgtagatgac tcgaagctg 19
<210> 13
   <211> 18
   <212> DNA
   <213> Artificial
<220>
   <223> reverse primer
<400> 13
   accatcctcg acaagcag 18
<210> 14
   <211> 18
   <212> DNA
   <213> Artificial
<220>
   <223> oligonucleotide
<400> 14
   gagaggcagg gaaagagg 18
<210> 15
   <211> 20
   <212> DNA
   <213> Artificial
<220>
   <223> forward primer
<400> 15
   ctcgatcgaa tccgtaaaca 20
<210> 16
   <211> 21
   <212> DNA
   <213> Artificial
<220>
   <223> reverse primer
<400> 16
   ccgcgtatgt tttatcgaga c 21
<210> 17
   <211> 17
   <212> DNA
   <213> Artificial
<220>
   <223> oligonucleotide
<400> 17
   atccttggac cgagcta 17
<210> 18
   <211> 17
   <212> DNA
   <213> Artificial
<220>
   <223> oligonucleotide
<400> 18
   atccttggac cgaactc 17
<210> 19
   <211> 20
   <212> DNA
   <213> Artificial
<220>
   <223> forward primer
<400> 19
   aggtccaaca tgcacagtga 20
<210> 20
   <211> 20
   <212> DNA
   <213> Artificial
<220>
   <223> reverse primer
<400> 20
   ggcaaagaaa atgtgggcta 20
<210> 21
   <211> 17
   <212> DNA
   <213> Artificial
<220>
   <223> oligonucleotide
<400> 21
   ggatacaaac tttggaa 17
<210> 22
   <211> 20
   <212> DNA
   <213> Artificial
<220>
   <223> forward primer; N is T or C
<400> 22
   agcgaattcg agatgctgnt 20
<210> 23
   <211> 20
   <212> DNA
   <213> Artificial
<220>
   <223> Reverse primer; N is T or G
<400> 23
   ccttnatctt gctgcgcttc 20
<210> 24
   <211> 16
   <212> DNA
   <213> Artificial
<220>
   <223> oligonucleotide
<400> 24
   caagctgacg gacatt 16
<210> 25
   <211> 16
   <212> DNA
   <213> Artificial
<220>
   <223> oligonucleotide
<400> 25
   caagctgacg gagatc 16
<210> 26
   <211> 20
   <212> DNA
   <213> Artificial
<220>
   <223> oligonucleotide
<400> 26
   ggtacaacat catcaacttc 20
<210> 27
   <211> 16
   <212> DNA
   <213> Artificial
<220>
   <223> oligonucleotide
<400> 27
   gggacatagg ccagag 16
<210> 28
   <211> 20
   <212> DNA
   <213> Artificial
<220>
   <223> oligonucleotide
<400> 28
   agaatccgta tctccatata 20
<210> 29
   <211> 20
   <212> DNA
   <213> Artificial
<220>
   <223> oligonucleotide
<400> 29
   cagaatccgt atctccatat 20

## Description

The present invention relates to a method and to a microarray for detecting herpesviruses.

Herpesviruses cause a number of human diseases of clinical importance. Especially diseases of the central nervous system (CNS) and complications in immunocomprimised patients can be the result of infection or reactivation by herpes simplex type 1 or 2 (HSV-1, HSV-2), cytomegalo- (CMV), Epstein-Barr (EBV), varicella zoster (VZV) or human herpes virus 6 (HHV-6) (Aurelius et al., 1991; Koskiniemi et al., 2002; Piiparinen et al. 2002, Aalto et al., 2003). In addition to HHV-6, human herpes virus 7 (HHV-7) has been reported to be a causative agent of exanthema subitum and documented to cause severe complications in transplant recipients (Tanaka et al., 1994; Suga et al., 1997). Virus isolation, nucleic acid detection and a variety of serological methods are currently used to detect herpesvirus infections (Chiu et al., 1998; Pitkäranta et al., 2000; Espy et al., 2000; Read et al., 2001; Ihira et al., 2002). Multiplex-PCR assays have been developed in order to meet the demand for fast detection of herpes viruses (Read and Kurtz, 1999; Aberle and Puchhammer-Stöckl, 2002; Druce et al., 2002; Hudnall et al., 2004). A single tube PCR-assay for simultaneous amplification of various herpes viruses is disclosed in Yamamoto and Nakamura (2000) and differentiation and quantitation of human herpesviruses by real-time PCR in Safronetz et al. (2003). US 2004/0110195 discloses a method for detecting and typing human herpesviruses involving the use of multiplex PCR assays and consensus primers to amplify conserved regions of herpesvirus DNA. US 20040053264 discloses a method for simultaneous detection of pathogenic organisms including herpesviruses by using a DNA chip. US 20030228599 discloses a multiplexed analysis technique for screening herpesviruses. US 20030143571 and Földes-Papp et al. (2004) disclose a method for simultaneous detection of a plurality of herpesviruses using microarray. Striebel et al. (2004) disclose studies concerning human herpesvirus diagnosis with DNA microarrays using dendrimers. In the publication of Boriskin et al (2004) microarrays were used in CNS infections to detect 13 different pathogens including several herpesviruses using long probes.

Although several methods for detecting various herpesviruses have been disclosed in the prior art, still new methods are needed by which the efficiency and speed of the detection of herpesviruses could be improved. Also only few methods make possible the simultaneous detection of several herpesviruses from the same biological sample. In particular, only some publications report of a method, which can make a distinction between HHV-6A and HHV-6B viruses.

Primers used to amplify some of the herpesviruses are known from US 6,897,057, JP 6253900 and Vesanen et al. (1996) Yamamoto and Nakamura (2000), Akhtar et al. (1996), Van den Veyver et al. (1998), WO 9325707, US 20040110195 and Saffronetz et al. (2003). Promoter sequence for T3 RNA polymerase used in the amplification of specific nucleic acids is known from JP2001095590, EP 510085 and US 2004/0125774.

### SUMMARY

It is an aim of the present invention to provide a new and improved method for detecting herpesviruses.

In particular, it is an aim of the present invention to provide an efficient and rapid method for the detection of one or more herpesviruses from the same biological sample and/or from different biological samples. More specifically, it is an aim of the present invention to provide a method, which makes possible efficient and rapid detection of several herpesviruses simultaneously.

It is another aim of the invention to provide a microarray for efficient and rapid detection of herpesviruses.

These and other objects, together with the advantages thereof over known methods and microarrays are achieved by the present invention, as hereinafter described and claimed.

The method of this invention is based on microarray technology. According to the method several different herpesviruses can be detected simultaneously from the same and/or different biological sample. Also several samples can be studied at the same time by the method. It is also possible to determine the genotype of the viruses in the same reaction.

According to an embodiment of the invention the method of the invention comprises the following steps:
- extracting DNA from a biological sample;
- amplifying the extracted DNA;
- translating the amplified DNA to ssRNA;
- hybridizing ssRNAs to oligonucleotide sequences on a microarray plate, said oligonucleotide sequences corresponding to each of the herpesviruses to be detected;
- extending the hybridised ssRNA by primer extension method in the presence of detectable nucleotides, and
- detecting a signal from the detectable nucleotides by a suitable method.

More specifically, the method is characterized by what is stated in claims 1 to 11 and 24.

According to another embodiment of the invention a microarray for detecting herpesviruses comprises:
- a solid support comprising at least one array; and
- said array comprising oligonucleotides specific for at least one herpesvirus.

More specifically, the microarray is characterized by what is stated in claims 12 to 17 and a method for preparing a microarray for detecting herpesviruses is characterized by what is stated in claim 19.

A microarray kit is characterized by what is stated in claim 18 and an oligonucleotide is characterized by what is stated in claim 20, a primer pair is characterized by what is stated in claim 21, and a set of multiplex primer pairs is characterized by what is stated in claims 22 and 23.

Diagnostic PCRs are commonly done for one herpesvirus at a time and this increases the detection time for several herpesviruses. Double and triple infections could be lost with PCRs whereas multiple infections may be detected by microarray in a day. The simultaneous detection of several herpesviruses is of great advantage. According to one preferred embodiment of the invention under suitable conditions the specificity of the microarray method of the present invention was found to be up to 100%. The microarray-based detection offers a fast and convenient protocol for detection of several herpesviruses for both diagnostics and research purposes. The method is in particular suitable for qualitative detection of herpesviruses.

### DESCRIPTION OF THE FIGURES

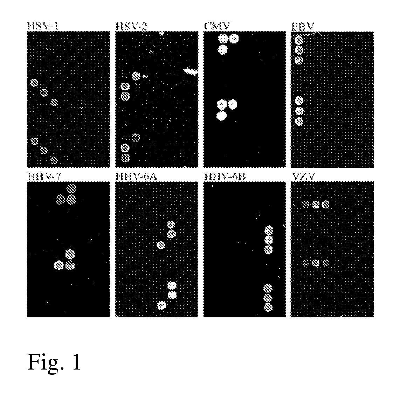

Fig. 1. Identification of eight herpesviruses by microarray-based method by using imaging. All the herpesvirus-specific oligonucleotides were spotted in 2×triplicate per array and unspecific oligonucleotides twice per array. Each array contained 60 spots (5×12 matrix). There were no signals observed from unspecific oligonucleotides. These images of HSV-1, HSV-2, CMV, EBV, HHV-7 and VZV were from average levels of viral DNA controls (range area 1000-5000 copies or VPs). In HHV-6A and -6B images no cross-reactions were observed in low levels of viral DNA (levels in image: 290 copies and 220 VPs, respectively).

### DETAILED DESCRIPTION OF THE INVENTION

"Herpesviruses" belong to family *Herpesviridae.* They are composed of a core of doublestranded DNA within an icosahedral capsid and a phopholipid bilayer.

Of the more than 100 known herpesviruses, eight infect humans. Human herpesviruses are classified into three subfamilies - alpha-, beta- and gamma-herpesviruses - on the basis of the length of viral replication cycle and host tissue range. Herpes simplex virus 1 (HSV-1), Herpes simplex virus 2 (HSV-2) and varicella-zoster virus (VZV) belong to subfamily alpha, cytomegalovirus (CMV), herpesvirus 6 (HHV-6) and herpesvirus 7 (HHV-7) belong to subfamily beta and Epstein-Barr virus (EBV) and herpesvirus (HHV-8) belong to subfamily gammaherpesviruses. Human infections are very common and patients can be infected with one or more herpesviruses, for example with both HSV-1 and HSV-2.

By the method of the present invention it is possible to detect one or more of a plurality of herpesviruses in a biological sample. Preferably the viruses are selected from the group comprising HSV-1, HSV-2, cytomegalo- (CMV), Epstein-Barr (EBV), varicella zoster (VZV), human herpes virus 6 (HHV-6A and HHV-6B) and human herpes virus 7 (HHV-7). In particular, by the method of the present invention can be made a distinction between HHV-6A and HHV-6B.

More specifically, by the method of the present infection it is possible to detect simultaneously at least two, preferable at least three, more preferably at least four different herpesviruses from the same sample.

According to one preferred embodiment of the invention herpesviruses classified to the same subfamily, alpha-, beta- or gamma, are detected by the method of the present invention simultaneously.

According to another preferred embodiment of the invention herpesviruses commonly causing a double or triple infection in a patient are detected by the method of the present invention simultaneously.

Furthermore, other pathogens commonly causing infection at the same time as herpesviruses may be detected simultaneously.

By "biological sample" is here meant any sample from biological origin. In particular the sample is a clinical sample from a subject, preferably from a human subject. The sample may comprise whole blood, plasma, cerebrospinal fluid (CSF) sample, biopsy, urine, stool, sputum, mucus, bone marrow, skin, hair, nails, a lesional or ulcerous swab, a cervical/vaginal swab, a nasopharyngeal swab, a bronchoalveolar lavage, an endotracheal aspirate or any other clinical sample. Preferred clinical samples are plasma, whole blood and cerebrospinal fluid (CSF) samples.

The method of the present invention comprises that DNA is extracted from the biological sample to be studied. DNA can be extracted from the biological sample by using well-known DNA extraction methods. In the extraction can be used for example commercially available kits, such as MagNA Pure LC instrument and Total Nucleic Acid Kit (Roche Diagnostics, Basel, Switzerland), High Pure Viral Nucleic Acid Kit (Roche Diagnostics) or chloroform-phenol extraction.

The extracted DNA can then be amplified by using a suitable method. Preferably the amplification method is a PCR (polymerase chain reaction) method. PCR refers to the method for increasing the concentration of a segment of a target sequence in a mixture of DNA without cloning or purification. This process for amplifying the target sequence consists of introducing a large excess of two oligonucleotide primers to the DNA mixture containing the target sequence, followed by a precise sequence of thermal cycling in the presence of a DNA polymerase. The two primers are complementary to their respective strands of the double stranded target sequence. In this case the target sequence is a herpes virus sequence to be detected. It may also be a sequence of another pathogen to be detected. Because the desired amplified segments of the target sequence become the predominant sequences (in terms of concentration) in the mixture, they are said to be "PCR amplified".

The primers for the PCR can be prepared according to methods well known in the art and described, e.g., in Sambrook, J. Fritsch, E. F., and Maniatis, T. (1989). Molecular Cloning: A Laboratory Manual, Cold Spring Harbor Laboratory Press, Cold Spring Harbor, N.Y.). The primer pair typically consists of a forward primer (sense) and reverse primer (antisense) corresponding to the specific areas of a herpesvirus sequence to be detected. The primer pair can be designed using commercially available software aimed for primer design, for example Primer3-software (http://frodo.wi.mit.edu/cgi-bin/primer3/primer3_www.cgi; Rozen and Skaletsky, 2000) and DNA mfold software (http://www.bioinfo.rpi.edu/applications/mfold/old/dna/; Zuker, 2003.

The amplification of the sample DNA can be made in one or several PCR amplification reactions. If more than one primer pair is used in the PCR, the method is called multiplex PCR. It is possible to amplify the DNA in one or more multiplex PCRs. For example here is disclosed the amplification of primer pairs for two or more herpesviruses in one multiplex PCR and primer pairs for two or more other herpesviruses in another multiplex PCR. If the DNA from one sample has been amplified in more than one PCR reaction, the PCR products from different reactions can be combined before further steps are carried out.

As is disclosed here a primer pair is designed for each herpesvirus to be detected. The primer pair comprises primer A and primer B, wherein advantageously
A is SEQ ID NO: 1 and B is SEQ ID NO: 3;
A is SEQ ID NO: 6 and B is SEQ ID NO: 7;
A is SEQ ID NO: 9 and B is SEQ ID NO: 10;
A is SEQ ID NO: 12 and B is SEQ ID NO: 13;
A is SEQ ID NO: 15 and B is SEQ ID NO:16; or
A is SEQ ID NO: 19 and B is SEQ ID NO: 20.

According to a preferred embodiment of the invention instead of SEQ ID NO:1 can be used SEQ ID NO: 22 and instead of SEQ ID NO:3 can be used SEQ ID NO: 23. These sequences can be used also in addition to the original sequences.

Primer A is preferably a forward primer and primer B is preferably a reverse primer.

It should be understood that equivalent primer sequences comprise sequences, which have at least 90 % identity, preferably at least 95 %, more preferably at least 97% identity to the above mentioned primer pair sequences A or B. More preferably the sequences have at least 99% identity, most preferably 100% identity to the mentioned sequences. In particular there may be differences in the 5' ends of the primers.

"Primer sequence having essentially the same sequence" refers to a primer sequence lacking one nucleotide, having one additional nucleotide, or having one change in the nucleotide sequence compared to the primer sequences SEQ ID NO: 1, SEQ ID NO: 3; SEQ ID NO: 6, SEQ ID NO: 7, SEQ ID NO: 9, SEQ ID NO:10, SEQ ID NO: 12, SEQ ID NO:13, SEQ ID NO: 15, SEQ ID NO:16, SEQ ID NO: 19, SEQ ID NO: 20, SEQ ID NO:22 or SEQ ID NO: 23. It may be also of advantage if there is nucleic acid variance, for example T/C, in primers (both primer alternatives available in the same mixture).

The primers as used herein refers to primers having a contiguous sequence of 10 to 50 nucleotides, preferably 20 to 40, more preferably 20 to 30 nucleotides. Primers used to perform the PCR reaction encompass nucleotide sequences of sufficient length and appropriate sequence so as to provide initiation of polymerization on a nucleic acid molecule.

In particular, primer pair SEQ ID NO: 1 and SEQ ID NO: 3 is designed for amplifying HSV-1 and HSV-2, primer pair SEQ ID NO: 6 and SEQ ID NO: 7 is designed for amplifying VZV, primer pair SEQ ID NO: 9 and SEQ ID NO:10 is designed for amplifying CMV, primer pair SEQ ID NO: 12 and SEQ ID NO:13 is designed for amplifying EBV, primer pair SEQ ID NO: 15 and SEQ ID NO:16 is designed for amplifying HHV-6, and primer pair SEQ ID NO: 19 and SEQ ID NO: 20 is designed for amplifying HHV-7. According to an alternative, preferred embodiment of the invention primer pair SEQ ID NO: 22 and SEQ ID NO: 23 is designed for amplifying HSV-1 and HSV-2.

The PCR amplification reaction is carried out under suitable conditions for the function of the DNA polymerase in the reaction mixture. The reaction mixture comprises DNA polymerase, all four nucleotide triphosphates (NTPs), a suitable buffer and optionally salts or other reagents. The amplification may consist for example of denaturation at about 95°C for 10 min, followed by 40 cycles at 93-97°C, preferably 96°C for 10 sec, 50-60 °C preferably 55°C for 20 sec and about 69 - 74 °C, preferably 72°C for 20 sec and final extension 69 - 74 °C, preferably 72°C for 5 min.

Alternatively, the amplification may consist for example of denaturation at about 95°C for 10 min, followed by 30 cycles at 93-97°C, preferably 96°C for 10 sec, 45-70 °C preferably 65°C for 20 sec (a 0.5°C decrease per cycle) and about 69 - 74 °C, preferably 72°C for 20 sec, 10 cycles at 93-97°C, preferably 96°C for 10 sec, 55-65 °C preferably 60°C for 20 sec and final extension 69 - 74 °C, preferably 72°C for 5 min.

The length of the amplified DNA (amplicons) is preferably between 150 to 250 bp, more preferably 170 to 230 bp.

Once the sample DNA has been amplified the products are preferably converted to single stranded nucleic acid, preferably transcribed to single stranded RNA. In order to facilitate this step of the procedure one of the primers of the primer pair can include polymerase promoter of the polymerase used in the reaction. According to this disclosure the promoter of T3 RNA polymerase was included to the reverse primers. If a polymerase promoter is included to the primer, the length of the primer may be doubled. According to this disclosure the primer for the promoter of T3 RNA polymerase preferably comprises SEQ ID NO:2. The transcribing reaction may be carried out by using commercially available kits, such as AmpliScribe™ T3 High Yield Transcription Kit (Epicentre, Madison, WI) or alternatively AmpliScribe™ T3-Flash™ Transcription Kit (Epicentre, Madison, WI).

Single stranded RNA is hybridised under suitable conditions to oligonucleotide sequences on a microarray plate, said oligonucleotide sequences corresponding to each of the herpesviruses to be detected.

As used herein "microarrays" refers to arrays comprising distinct oligonucleotides synthesized on a substrate, such as glass slide, silicon, plastic, a polymer matrix or any other suitable solid support. The microarray can be prepared and used according methods well-known for a person skilled in the art. The solid support may be e.g. a microscope glass slide or a silane-coated glass. The glass slide is preferably coated by isothiocyanate in order that aminated DNA-oligonucleotides can covalently bind to the glass (Lindroos et al. 2001).

Oligonucleotides for herpesviruses can be designed by using a suitable software, such as Primer3-software (http://frodo.wi.mit.edu/cgi-bin/primer3/primer3_www.cgi; Rozen and Skaletsky, 2000) and DNA mfold software (http://www.bioinfo.rpi.edu/applications/ mfold/old/dna/; Zuker. The oligonucleotides are attached to the solid support by a suitable method. The oligonucleotides can e.g. be modified to comprise amino group at their 5'end which can bind covalently to the silane - isothiocyanate layer on the solid support. The oligonucleotide may attach via its 5' terminus to 3'-amino-propyltrimethoxysilane +1,4-phenylenedi-isothiocyanate-coated glass surface by formation of a covalent bond. The oligonucleotides comprise preferably also a poly (T) primer (T spacer) at their 5'end before the specific oligonucleotide sequence. The length of the T spacer may be 6 to 12, preferably 8 to 10, typically 9 nucleotides. Also commercially available microarray glass slides having a suitable chemical surface, such as Asper Biotech, SAL-1 microarray slides, can be used.

The oligonucleotides as used herein refers to oligonucleotides having a contiguous sequence of about 10-50 nucleotides in length, preferably about 15-30, typically shorter than 30 nucleotides. More preferably the oligonucleotides are about 15 - 25 nucleotides in length, most preferably 16 to 23. Typically they may be about 20 nucleotides in length.

Oligonucleotides attached to the microarray encompass nucleotide sequences of sufficient length and appropriate sequence so as to provide hybridization of ssRNA of the tested herpes viruses in the sample.

Oligonucleotides may be spotted on the solid support by a suitable method, for example by a commercially available microarrayer such as OmniGrid®, GeneMachines, Huntingdon, UK. The array may consist of a matrix comprising the oligonucleotides for herpesviruses to be detected. The array may consist also of oligonucleotides with sequences unspecific for viruses as a control. In addition the array may consist also of oligonucleotides with sequences specific for other pathogens than herpesviruses. The solid support may contain 1 to 80, preferably 10 to 60 arrays. This means that on the same solid support can be studied simultaneously up to 80 samples.

The number of spots on an array is 4 to 200, preferably 10 to 100, more preferably 20 to 50, typically 30 to 40. The spots may consist of the oligonucleotides in duplicate or triplicate. They may consist of e.g. 10 to 25, typically 15 - 20 oligonucleotides in duplicate.

The amount of oligonucleotide per spot may be 0.5 to 1.5 pmol, typically about 1 pmol.

There may be about 200 spots per one array and the number of spots per one solid support, for example a slide may be about 16 000.

The ssRNA is hybridised to the oligonucleotides and a primer extention reaction is carried out under suitable conditions. The hybridisation temperature may be 40 to 45 C°, preferably about 42 C°. The hybridisation time may be about 15 to 30 minutes. The hybridisation may be carried out in varying salt concentrations, for example in varying amounts of NaCl.

The primer extension reaction is carried out in the presence of a reverse transcriptase enzyme and nucleotide triphosphates, optionally with suitable buffers, salts and other reagents. The reaction may be carried out at the temperature of at least 42 °C, preferably at the 49 to 55 °C, more preferably at the temperature of about 52°C. The reaction may be carried out in 15 to 30 min, preferably about 20 min. Preferably the reaction mixture comprises detectable nucleotides, such as fluorescent nucleotides, the signal of which can be detected by a suitable method.

The detectable nucleotide may comprise any detectable reporter or signal moiety including, but not limited to radioisotopes, enzymes, antigens, antibodies, spectrophotometric reagents, chemiluminescent reagents, fluorescent and any other light producing chemicals.

The microarrays may be analysed by a suitable method, such as ScanArray Express scanner, ScanArray^{™} and QuantArray^{™} software (PerkinElmer, Wellesley, MA). The signal of the oligonucleotide spot (minus local background signal) may be compared to signals of unspecific oligonucleotides. The level or amount of signals is adjusted for positive and negative result. According to this disclosure signals of at least three times higher were considered positive.

The oligonucleotides preferably comprise a nucleotide sequence selected from the group of sequences:

The SEQ ID NO:4, SEQ ID NO: 5, SEQ ID NO:8, SEQ ID NO:11, SEQ ID NO: 14, SEQ ID NO: 17, SEQ ID NO: 18 and SEQ ID NO: 21. According to a preferred embodiment of the invention instead of sequence SEQ ID NO:4 can be used SEQ ID NO:24, instead of sequence SEQ ID NO:5 can be used SEQ ID NO:25 or general oligonucleotide sequences SEQ ID NO:26 or SEQ ID NO:27 or several of them and instead of sequence SEQ ID NO :8 can be used sequences SEQ ID NO:28 or SEQ ID NO:29 or both of them. The alternative oligonucleotide sequences can be used also in addition to (together with) the original oligonucleotide sequences.

It should be understood that equivalent oligonucleotide sequences comprise sequences, which have at least 90 % identity, preferably at least 95 %, more preferably at least 97 % identity to the sequences SEQ ID NO:4, SEQ ID NO: 5, SEQ ID NO:8, SEQ ID NO: 11, SEQ ID NO: 14, SEQ ID NO: 17, SEQ ID NO: 18, SEQ ID NO: 21, SEQ ID NO: 24, SEQ ID NO: 25, SEQ ID NO: 26, SEQ ID NO: 27, SEQ ID NO: 28 or SEQ ID NO: 29. More preferably the sequences have at least 99% identity, most preferably 100% identity to the mentioned sequences. If there are differences in the sequence, the differences may preferably be in the 5'end of the oligonucleotide.

"Oligonucleotide sequence having essentially the same sequence" refers to an oligonucleotide sequence comprising a sequence lacking one nucleotide, having one additional nucleotide, or having one change in the nucleotide sequence compared to the sequences SEQ ID NO:4, SEQ ID NO: 5, SEQ ID NO:8, SEQ ID NO: 11, SEQ ID NO: 14, SEQ ID NO: 17, SEQ ID NO: 18, SEQ ID NO: 21, SEQ ID NO: 24, SEQ ID NO: 25, SEQ ID NO: 26, SEQ ID NO: 27, SEQ ID NO: 28 or SEQ ID NO: 29.

Additional nucleotides may be added to either end of the above defined sequence of the oligonucleotide. However, this may result in secondary structures, which may cause less successful hybridisation. The oligonucleotides may be designed so that their melting temperature, Tm, is 49 to 54 °C. Depending on the temperature, there should be a smaller or higher number of free nucleotides at the 5'end of the oligonucleotide. According to this disclosure the 5'end comprises a T spacer. The 5'end of the oligonucleotide may comprise in addition to the T spacer 1 or 2 additional nucleotides . However, it is of advantage, if the 5 'end does not comprise any additional nucleotides. The 5'end may also lack 1 or 2 nucleotides. However, it is also of advantage, if the 5'end does not lack any nucleotide.

According to a preferred embodiment of the invention the microarray comprises oligonucleotides corresponding to at least two, preferably at least three different herpesviruses, more preferably at least four different herpesviruses. According to a preferred embodiment of the invention the microarray comprises oligonucleotides corresponding to 2 to 8 different herpesviruses. According to most preferred embodiments, the microarray comprises oligonucleotides corresponding to at least five, preferably at least six, more preferably at least seven or at least eight different herpesviruses. Only a few publications describe methods, by which the detection of seven or eight different herpes viruses is possible. Very few methods make possible the detection of HHV-6A and HHV-6B viruses. It is of particular advantage that all of the mentioned 8 herpesviruses can be detected simultaneously from the same biological sample.

According to one preferred embodiment of the invention at least oligonucleotides comprising sequences selected from the group comprising SEQ ID NO:4 and SEQ ID NO: 5 or at least oligonucleotides comprising sequences selected from the group comprising SEQ ID NO:4, SEQ ID NO: 5 and SEQ ID NO: 8 are used to detect herpesviruses simultaneously.

Alternatively, at least oligonucleotides comprising sequences selected from the group comprising SEQ ID NO:24, SEQ ID NO: 25, SEQ ID NO:26 and SEQ ID NO:27 or at least oligonucleotides comprising sequences selected from the group comprising SEQ ID NO:24, SEQ ID NO: 25, SEQ ID NO:26, SEQ ID NO:27, SEQ ID NO:28 and SEQ ID NO:29.

According to another preferred embodiment of the invention at least oligonucleotides comprising sequences selected from the group comprising SEQ ID NO:11 and SEQ ID NO: 17 and SEQ ID NO: 18, or at least oligonucleotides comprising sequences selected from the group comprising SEQ ID NO:11, SEQ ID NO: 17, SEQ ID NO: 18 and SEQ ID NO 21 are used to detect herpesviruses simultaneously.

According to one further preferred embodiment of the invention oligonucleotides comprising sequences detecting herpesviruses commonly causing a double or triple infection in a patient are detected by the method of the present invention simultaneously.

According to another further preferred embodiment of the invention the method comprises the detection of other pathogens or other viruses from the same biological sample. The other pathogens or viruses may be those commonly causing infection at the same time as herpesviruses. Alternatively, the other pathogens or viruses may be those causing central nervous system disorders or immune deficiency diseases. Examples of possible other pathogens are enteroviruses or borrelia causing pathogens.

The present invention provides also a microarray kit, which comprises the microarray and optionally reagents needed in the reaction. Such reagents are for example enzymes, nucleotide triphosphates (NTPs) and buffers.

By "qPCRs" is here meant quantitative PCR.

By "qualitative PCR" is meant here diagnostic PCR.

The method of the present invention has been exemplified here by designating a microarray for the detection of eight herpesviruses. Microarray-based detection was compared to every-day diagnostic PCRs. Every-day diagnostic PCRs were exemplified by preparing two multiplex-PCRs.

The clinical specimens represented true patient cases. Concordant results from diagnostic PCRs and microarray were obtained in 89%. Microarray gave positive result in four cases whereas diagnostic PCRs remained negative.

There were two double infections (CMV-EBV) among CMV-positive plasma specimens detected by microarray. These specimens were from renal and bone marrow transplant patients. Both patients were seropositive for EBV and CMV. Among EBV-positive specimens we detected two double infections (EBV-CMV and EBV-HHV-7) with microarray. The EBV-CMV co-infection was a bone marrow transplanted patient, who was seropositive for EBV and had CMV IgM and IgG antibodies. In the case of EBV-HHV-7 infection the sample from a renal transplant patient was seronegative for HHV-7.

Two VZV-positive CSF samples were VZV-HHV-6B and VZV-EBV positive by microarray. Earlier studies had shown that the VZV-HHV-6B positive patient had also intrathecal antibodies against VZV and HHV-6. In the possible VZV-EBV co-infection, VZV IgG was verified in serum. One possible triple infection (HSV-2, HHV-7 and HHV-6B) was found by microarray. The sample was seronegative for HHV-7 and tested negative against HSV-1 and -2 by diagnostic PCR.

CSF cell counts, protein and glucose values were available for only 3 VZV-positive and 1 HSV-2-positive (possible triple infection) patients. In two of these, the findings were normal, whereas the other two showed elevated leukocyte and erythrocyte counts, normal glucose values and elevated protein levels. The HSV-2-positive patient had also HSV-IgM antibodies in CSF.

In diagnostics HHV-7 is not tested from clinical specimens by PCR. Four specimens, that were HHV-7 positive by microarray, were seronegative for HHV-7 at the time of sampling. Therefore, acute infections cannot be ruled out. HHV-7 is quite commonly found, e.g. in transplant patients (Dockrell et al, 2001).

Diagnostic PCRs are commonly done for one herpesvirus at a time and this increases the detection time for several herpesviruses. Double and triple infections could be lost with PCRs whereas multiple infections may be detected by microarray in a day. We conclude that microarray-based detection offers a fast and convenient protocol for detection of several herpesviruses for both diagnostics and research purposes.

### Examples

### Example 1

### Viral DNA and clinical specimens

In setting up of multiplex-PCRs and microarray, serial 10-fold dilutions of commercial viral DNA controls (started from million copies or virus particles) and cell culture supernatants were used (Table I).

**TABLE I. List of commercial viral DNA controls (Autogen Bioclear, Wiltshire, UK) and cell supernatants used for optimization and testing of multiplex-PCR and microarray.**

| Virus | Strain | Quality of sample |
|---|---|---|
| HSV-1 | MacIntyre | commercial viral DNA |
| HSV-2 | G | |
| CMV | AD169 | |
| EBV | B95-8 | |
| HHV-7 | H7-4 | |
| HHV-6A | U1102 | |
| HHV-6B | Z-29 | |
| VZV | Rodstrain | |
| HHV-6A | house control* GS | cell supernatant |
| HHV-6B | Z-29 | |

| | | |
|---|---|---|
| * Clincal isolate used as a house control. | | |

In total, 116 clinical specimens were collected from pretested clinical material containing plasma (73), whole blood (10), cerebrospinal fluid (CSF) samples (23) and proficiency-testing samples (10) (QCMD, Glasgow, Scotland, UK). Plasma, whole blood and CSF samples were collected from solid organ and bone marrow transplant patients, and patients with neurological symptoms. Of the proficiency-testing samples, 4/10 were derived from a VZV and 6/10 from an HSV proficiency program 2004 (Table II). To study the specificity of the microarray, we tested 70 sera from patients (suspected epidemic nephropathy), 30 CSFs tested negative for HSV, EBV, CMV, VZV, HHV-6 and -7 DNA and 11 samples (Table II) from an enterovirus proficiency program 2004.

**TABLE II. Virus proficiency programs, strains, viral DNA loads and panel formats of proficiency testing samples used for testing of microarray.**

| Virus proficiency program | Strain | Viral DNA load (GEq/ml) | Panel format |
|---|---|---|---|
| Varicella-zoster virus, 2004 | VZV strain 9/84 SMI | 700 | Lyophilized cultured VZV |
| | | 70 000 | |
| | | 7 000 000 | |
| | HSV-1 ATCC strain MacIntyre | 210 000 000 | |
| Herpes simplex virus, 2004 | HSV-1 ATCC strain MacIntyre | 24000 | Freeze dried inactived samples |
| | HSV-2 ATCC strain MS | 1500 | |
| | | 15 000 | |
| | | 15 000 000 | |
| | VZV strain 9/84 SMI | 500 000 | |
| | neg | - | |
| Enterovirus, 2004* | selected EV serotypes | - | Lyophilized cultured EV |

| | | | |
|---|---|---|---|
| GEq/ml, genome equivalent per ml; neg, negative; EV, enterovirus. All proficiency testing samples were diluted in sterile water (1ml). * Eleven enteroviral proficiency testing samples contained enteroviruses (8/11), rhinoviruses. (1/11) and 2 were negative. | | | |

### DNA extraction

DNA was extracted using MagNA Pure LC instrument and Total Nucleic Acid Kit (Roche Diagnostics, Basel, Switzerland), High Pure Viral Nucleic Acid Kit (Roche Diagnostics) or chloroform-phenol extraction depending on the protocol used in diagnostics.

### Multiplex-PCR primers and oligonucleotides

The primer pair for HSV-1 and -2 (multiplex-PCR1) was modified from published primers (Piiparinen and Vaheri, 1991). Oligonucleotides and primers for CMV, EBV, VZV, HHV-6A, -6B and HHV-7 (multiplex-PCR2) were designed using Primer3-software (http://frodo.wi.mit.edu/cgi-bin/primer3/primer3 www.cgi; Rozen and Skaletsky, 2000) and DNA mfold software (http://www.bioinfo.rpi.edu/applications/mfold/old/dna/; Zuker, 2003). T3 RNA polymerase promoter sequence was included in the reverse multiplex-PCR primers (Tables III and IV).

Alternatively oligonucleotides and primers for HSV-1, HSV-2, CMV, EBV, VZV, HHV-6A, -6B and HHV-7 (multiplex-PCR1 and -2) were designed using Primer3-software (http://frodo.wi.mit.edu/cgi-bin/primer3/primer3_www.cgi; Rozen and Skaletsky, 2000) and DNA mfold software (http://www.bioinfo.rpi.edu/applications/mfold/old/dna/; Zuker, 2003). Alternative sequences in Table III are SEQ ID NO:22 to 26. Alternative sequences in Table IV are SEQ ID NO:28 and 29.

**TABLE III. Primers used for multiplex-PCR1 and oligonucleotides on microarray.**

| Genus, GenBank accession No. | Oligonucleotides | Gene name | Sequences (5'→ 3') | Position |
|---|---|---|---|---|
| HSV-1, X14112 | HSV-FW | DNA pol., UL30 | AAGGAGGCGCCCAAGCGCCCG (SEQ ID NO:1) | 64750 - 64768 |
| | | | AGCGAATTCGAGATGCTG(T/C)T (SEQ ID NO:22) | 64130-64149 |
| | HSV-RV | | AATTAACCCTCACTAAAGGGAGA (SEQ ID NO:2) | |
| | | | TGGGGTACAGGCTGGCAAAGT (SEQ ID NO:3) | 64976 - 64956 |
| | | | CCTT(T/G)ATCTTGCTGCGCTTC (SEQ ID NO:23) | 64355-64336 |
| | HSV-1-T3 | | Am-TTTTTTTTTGTCCTTGACCCCACTT (spacer + SEQ ID NO:4) | 64907 - 64922 |
| | | | Am-TTTTTTTTTCAAGCTGACGGACATT (spacer + SEQ ID NO:24) | 64237-64252 |
| HSV-2, Z86099 | HSV-FW | DNA pol., UL30 | AAGGAGGCGCCCAAGCGCCCG (SEQ ID NO:1) | 65209 - 65229 |
| | | | AGCGAATTCGAGATGCTG(T/C)T (SEQ ID NO:22) | 64591-64610 |
| | HSV-RV | | AATTAACCCTCACTAAAGGGAGA (SEQ ID NO:2) | |
| | | | TGGGGTACAGGCTGGCAAAGT (SEQ ID NO:3) | 65449 - 65429 |
| | | | CCTT(T/G)ATCTTGCTGCGCTTC (SEQ ID NO:23 | 64816-64797 |
| | HSV-2-T3 | | Am-TTTTTTTTTAGGATAAGGACGACGAC (SEQ ID NO:5) | 65279 - 65295 |
| | | | Am-TTTTTTTTTCAAGCTGACGGAGATC (SEQ ID NO:25) | 64698-64713 |
| HSV (HSV-1, X14112 ; HSV-2, Z86099) | HSV-GEN1-T3 | | Am-TTTTTTTTTGGTACAACATCATCAACTTC (SEQ ID NO:26) | HSV-1: 64197-64216; HSV-2: 64658-64677 |
| | HSV-GEN2-T3 | | Am-TTTTTTTTTGGGACATAGGCCAGAG (SEQ ID NO:27) | HSV-1: 64311-64326; HSV-2: 64722-64787 |

| | | | | |
|---|---|---|---|---|
| FW, forward primer (sense); RV, reverse primer (anti-sense, T3 RNA polymerase promoter sequence AATTAACCCTCACTAAAGGGAGA before virus sequence); T3, oligonucleotide (sense, 9×T spacer arm before sequence); Am, amino-link. Nucleic acid pair in parenthesis, such as for example Hsv-fw (T/C),means that there is nucleic acid variance in primers, i.e. there are both primer alternatives available in the same mixture. GEN, general (oligo for both HSV:s). | | | | |

**TABLE IV. Primers used for multiplex-PCR2 and oligonucleotides on microarray.**

| Genus, GenBauk accession No. | Oligonucleotides | Gene name | Sequences (5'→ 3') | Position |
|---|---|---|---|---|
| VZV, AY548171 | VZV-FW | DNA pol., ORF28 | CCATTTTCTCGCCGATTTTA (SEQ ID NO:6) | 48508 - 48527 |
| | VZV-RV | | AATTAACCCTCACTAAAGGGAGA (SEQ ID NO:2) | |
| | | | GCCGCATTTGAACGTTTTAT (SEQ ID NO:7) | 48670 - 48651 |
| | VZV-T3 | | Am-TTTTTTTTTACCTCGTACGCTTTTTG (spacer + SEQ ID NO:8) | 48597 - 48613 |
| | VZV1-T3 | | Am-TTTTTTTTTAGAATCCGTATCTCCATATA (SEQ ID NO:28 | 48569-48588 |
| | VZV2-T3 | | Am-TTTTTTTTTCAGAATCCGTATCTCCATAT (SEQ ID NO:29 | 48568-48587 |
| CMV, AY446894 | CMV-FW | DNA pol., UL44 | GTACAACAGCGTGTCGTGCT (SEQ ID NO:9) | 57044 - 57063 |
| | CMV-RV | | AATTAACCCTCACTAAAGGGAGA (SEQ ID NO:2) | |
| | | | CACCGGCCATCAAGTTTATC (SEQ ID NO: 10) | 57240 - 57221 |
| | CMV-T3 | | Am-TTTTTTTTTGTAGAAGTTCTTCAGCTGC (spacer +SEQ ID NO:11) | 57122 - 57140 |
| EBV, AJ507799 | EBV-FW | DNA pol., BALF5 | CGTAGATGACTCGAAGCTG (SEQ ID NO:12) | 154029 - 154047 |
| | EBV-RV | | AATTAACCCTCACTAAAGGGAGA (SEQ ID NO:2) | |
| | | | ACCATCCTCGACAAGCAG (SEQ ID NO:13) | 154278 - 154261 |
| | EBV-T3 | | Am-TTTTTTTTTGAGAGGCAGGGAAAGAGG (spacer + SEQ ID NO: 14) | 154184- 154201 |
| HHV-6A, X83413 | HHV-6-FW | DNA pol., U38 | CTCGATCGAATCCGTAAACA (SEQ ID NO:15) | 59289 - 59308 |
| | HHV-6-RV | | AATTAACCCTCACTAAAGGGAGA (SEQ ID NO:2) | |
| | | | | 59444 - 59424 |
| | | | CCGCGTATGTTTTATCGAGAC (SEQ ID NO:16) | |
| | HHV-6A-T3 | | Am-TTTTTTTTTATCCTTGGACCGAGCTA (spacer + SEQ ID NO:17) | 59361 - 59377 |
| HHV-6B, AF157706 | HHV-6-FW | DNA pol., U38 | CTCGATCGAATCCGTAAACA (SEQ ID NO: 15) | 60409 - 60428 |
| | HHV-6-RV | | AATTAACCCTCACTAAAGGGAGA (SEQ ID NO:2) | |
| | | | CCGCGTATGTTTTATCGAGAC (SEQ ID NO:16) | 60564 - 60544 |
| | HHV-6B-T3 | | Am-TTTTTTTTTATCCTTGGACCGAACTC (spacer + (SEQ ID NO:18) | 60481 - 60497 |
| HHV-7, AF037218 | HHV-7-FW | DNA pol., U38 | AGGTCCAACATGCACAGTGA (SEQ ID NO:19) | 56787 - 56806 |
| | HHV-7-RV | | AATTAACCCTCACTAAAGGGAGA (SEQ ID NO:2) | |
| | | | GGCAAAGAAAATGTGGGCTA (SEQ ID NO:20) | 56993 - 56974 |
| | HHV-7-T3 | | Am-TTTTTTTTTGGATACAAACTTTGGAA (spacer + (SEQ ID NO:21) | 56893 - 56909 |

| | | | | |
|---|---|---|---|---|
| FW, forward primer (sense); RV, reverse primer (anti-sense, T3 RNA polymerase promoter sequence AATTAACCCTCACTAAAGGGAGA before virus sequence); T3, oligonucleotide (sense, 9×T spacer arm before sequence); Am, amino-link. | | | | |

### Multiplex-PCR amplification

Multiplex-PCR1 was used to identify HSVs resulting in a 264-bp amplicon and in the alternative method 249-bp amplicon.. Multiplex-PCR2 contained primer pairs for amplification of CMV, EBV, VZV, HHV-6A, -6B and -7 resulting in 220 bp, 273 bp, 186 bp, 179 bp (both HHV-6A and -6B) and 230 bp amplicons, respectively. Both multiplex-PCRs were carried out for each sample.

The multiplex-PCR1 was carried out in a final volume of 50 µl containing 50 mM KC1, 10 mM Tris-HCl (pH 8.3), 0.01% (w/vol) gelatine, 0.2 mM dNTPmix (Finnzymes, Espoo, Finland), 0.6 µM of primers, 12.5 U AmpliTaq Gold polymerase (Applied Biosystems, Foster City, CA) and 2 mM MgCl₂.

The multiplex-PCR2 was performed in a final volume of 53 µl containing 47.2 mM KC1, 9.4 mM Tris-HCl (pH 8.3), 0.009% (w/vol) gelatine, 0.19 mM dNTPmix (Finnzymes), 0.56 µM of each primers, 12.5 U AmpliTaq Gold polymerase (Applied Biosystems) and 1.9 mM MgCl₂. The amplification consisted of denaturation at 95°C for 10 min, followed by 40 cycles at 96°C for 10 sec, 55°C for 20 sec and 72°C for 20 sec and final extension 72°C for 5 min.

### PCRs in every-day diagnostics

Two real-time quantitative PCRs (qPCRs) for CMV and EBV, and three qualitative PCRs for HSVs, HHV-6, and VZV were used in our every-day diagnostics. One of these PCRs and microarray-based method were performed in parallel for the clinical specimens using DNA from the same extraction and the results were compared qualitatively. The choice of the used diagnostic PCR was based on the primary diagnostic testing result and clinical information.

qPCRs for CMV and EBV were performed according to Piiparinen et al (2004) and a modification from Aalto et al (2003), respectively. In the latter, EBV primers, and probe designed by Kimura et al (1999) were used. The PCR for HSVs was performed according to Piiparinen et al (1991) using the probes reported by Vesanen et al (1996). PCR for HHV-6 was done using the primers designed by Gopal (1990) and the HHV-6 probe reported by Pitkäranta et al (2000). PCR for VZV was done according to Echevarria et al (1994) and Koskiniemi et al (1997). Microplate hybridization with luminometric detection (Vesanen et al, 1996) was used for the detection of qualitative PCR products.

### Preparation of microarray

Microscope glass slides were activated as described by Guo et al (1994) with modification according to Pastinen et al (2000). The NH₂-modified oligonucleotides (Proligo, Paris, France) were spotted on slides using microarrayer (OmniGrid®, GeneMachines, Huntingdon, UK). The array consisted of a 5×12 matrix including eight oligonucleotides for herpesviruses (Table III and IV) and six oligonucleotides with sequences unspecific for viruses. The coated slides contained 50 arrays in which the herpesvirus-specific oligonucleotides were spotted in 2×triplicate and unspecific oligonucleotides functioning as negative hybridization controls twice per array. The spotting solution contained 20 µM oligonucleotide and 0.3 M sodium carbonate buffer (pH 9.0) or alternatively the commercial 1 × microarray spotting solution (MSS, Arraylt, Telechem International, Sunnyvale, CA) in a final volume of 10 µl. The spotting was performed at room temperature and 50% humidity. Slides were stored overnight at room temperature before use.

### In vitro RNA transcription and microarray reactions

Two multiplex-PCR products were pooled before transcribing into ssRNA using AmpliScribe™ T3 High Yield Transcription Kit (Epicentre, Madison, WI) or alternatively AmpliScribe™ T3 Flash Transcription Kit, 1 h 42°C) following the manufacturer's instruction. Negative controls of extractions, multiplex-PCRs and PCRs were included.

Mini Pap Pen (Zymed, South Francisco, CA) was used to encircle the array area. The ssRNA solution containing 6 µl ssRNA and 1.5 mM NaCl in a final volume of 8.5 µl was heated at 95°C for 1.5 min and allowed to react for 20 min at 42°C with array. Alternatively the ssRNA 6 µl was heated at 96°C for 2 min. After heating the ssRNA solution containing ssRNA 6 µl and 1.5 mM NaCl in a final volume of 8.5 µl was allowed to react for 20 min at 42°C with array. After incubation the microarrays were washed with array washing buffer [0.5×TE (5 mM Tris, 0.5 mM EDTA), 0.3 M NaCl and 0.1% Triton X-100 (YA Kemia, Helsinki, Finland)] and sterile water.

The primer extension solution in a final volume of 4.5 µl containing 55 mM Tris-HCl, 11 mM MgCl₂, 83 mM KC1, 11 mM DTT (Epicentre), 0.6 µM dATP, dGTP, ddATP, ddGTP, dUTP-CY5 and dCTP-CY5 (Amersham Bioscience, Little Chalfont, UK), 5 U MMLV reverse transcriptase (Epicentre) and 0.5 M trehalose (Sigma-Aldrich) and 8.3% glycerol (MP Biomedicals, Irvine, CA) was added and allowed to react for 20 min at 52°C. Before scanning fluorescence, the microarrays were washed with array washing buffer and dried.

### Scanning and analyzing the microarray

The microarrays were analyzed using a ScanArray Express scanner, ScanArray^{™} and QuantArray^{™} software (PerkinElmer, Wellesley, MA). The analyzing cut-off value was determined for each array separately. The signal of the oligonucleotide spot (minus local background signal) were compared to signals of unspecific oligonucleotides. Signals at least three times more were considered positive. The microarray was carried out in duplicate. The number of quantified signals did not correlate to number of virus in specimens.

### Analytical sensitivity

Multiplex-PCRs provided good analytical sensitivities (Table V) which were obtained by studying dilutions of commercial controls for each herpesvirus at minimum three times. Water controls of extractions and multiplex-PCRs were negative by microarray. Other cross-reactions than that between HHV-6A and -6B were not observed. At over 10 000 HHV-6B copies/reaction cross-reactions between genotype-specific oligonucleotides were observed. In high concentration of HHV-6A, however, no cross-reactions were detected. Commercial controls worked properly in detection (Fig. 1) and furthermore showed the accuracy of the hybridization.

**TABLE V. Sensitivities of multiplex-PCRs and microarray using commercial viral DNA controls.**

| Virus | Sensitivities of multiplex-PCRs per reaction | Sensitivity of microarray per reaction |
|---|---|---|
| HSV-1 | 9.1 VPs or | 9.1 VPs |
| | | |
| | 30 copies* | 5 copies* |
| HSV-2 | 8.0 VPs or | 8.0 VPs or 5 copies* |
| | 30 copies* | |
| CMV | 3.5 copies | 1.0 copies |
| EBV | 10.0 copies | 3.0 copies |
| HHV-7 | 10.0 copies | 3.0 copies |
| HHV-6A | 29.0 copies | 7.0 copies |
| HHV-6B | 22.0 VPs | 2.5 VPs |
| VZV | 11.0 VPs or 10 | 4.5VPs or 7.5 |
| | copies* | copies* |

| | | |
|---|---|---|
| VPs, virus particles. Sensitivities of multiplex-PCRs were defined by 2% gel electrophoresis which is less sensitive than hybridization methods. * indicates result received by the alternative method, oligonucleotides and/or primers. | | |

### Analysis of clinical specimens and specificity

Altogether 116 specimens were tested simultaneously using one of the diagnostic PCRs and microarray. qPCR for CMV or EBV was performed for 73 plasma and qualitative HHV-6-PCR for 10 whole blood samples (Table VI). Qualitative HSV- or VZV-PCR was run for 23 CSFs (Table VII). Ten proficiency-testing specimens (Table VIII) were tested with qualitative HSV- and VZV-PCR. For 103 out of 116 (89%) clinical specimens, PCRs and microarray gave concordant qualitative results.

**TABLE VI. Every-day diagnostic PCR and microarray results of plasma and whole blood specimens.**

| PCR result (number of specimens) | ¹Microarray result (number of specimens) |
|---|---|
| neg (32) | neg (28) |
| | CMV (1) |
| | EBV (1) |
| | HHV-6B (2) |
| CMV (26) | CMV (22) |
| | CMV and EBV (2) |
| | neg (2) |
| EBV (15) | EBV (12) |
| | EBV and CMV (1) |
| | EBV and HHV-7 (1) |
| | neg (1) |
| *HHV-6 (10) | HHV-6B (5) |
| | HHV-6B and EBV (2) |
| | HHV-6B and HHV-7 (2) |
| | neg (1) |

| | |
|---|---|
| neg, negative. Specimens were stored at -70 °C. * whole blood specimens ¹ Multiplex-PCRs detected by microarray. | |

Out of 32 PCR-negative plasma specimens, microarray was negative in 28 (Table VI). Four originally negative plasma specimens showed positive results with microarray. In 24/26 CMV-positive plasma specimens, qPCR and microarray gave concordant results. Two CMV-qPCR-positive samples, 1280 and 810 genome equivalents (GEq) per ml, remained negative by microarray. Two double infections of CMV and EBV were observed among CMV-positive cases (5420 GEq/ml for CMV and 6280 for EBV, and 4200 for CMV and 4130 EBV, respectively).

EBV was detected by microarray in all but one of 15 EBV-PCR-positive samples. The missed specimen gave 860 GEq/ml in qPCR. Two double infections (EBV-CMV and EBV-HHV-7) were detected with microarray. The specimens had 15×10⁶ and 990 GEq/ml for EBV in qPCR, respectively.

For 9 out of 10 HHV-6-PCR-positive specimens, HHV-6B was detected by microarray. Microarray detected also four double infections; two were HHV-6B-EBV, and the other HHV-6B-HHV-7 infections.

Altogether 10/23 CSF samples were negative and 13/23 positive by PCR. All PCR-negative CSFs were negative in microarray. Of 3 HSV-positive specimens microarray missed one HSV-2 positive sample. One triple infection (HSV-2, HHV-7 and HHV-6B) was observed by microarray. Both HHV-6-positive samples remained negative, whereas all 8 VZV-positive samples were positive also by microarray (Table VII).

**TABLE VII. Every-day diagnostic PCR and microarray results of CSF specimens*.**

| PCR result (number of specimens) | Microarray result (number of specimens) |
|---|---|
| neg (10) | neg (10) |
| HHV-6 (2) | neg (2) |
| HSV-1 (1) | HSV-1 (1) |
| HSV-2 (2) | HSV-2, HHV-7 and HHV-6B (1) |
| | neg (1) |
| VZV (8) | VZV (6) |
| | VZV and HHV-6B (1) |
| | VZV and EBV (1) |

| | |
|---|---|
| neg, negative. Specimens stored at -70 °C. | |

Two out of 10 proficiency-testing specimens were negative by PCR and microarray. Of eight PCR-positive specimens, two were positive for HSV-1, two for HSV-2, and four for VZV, as was previously been informed by the QCMD. Microarray remained negative in one of the HSV-1 and -2-PCR-positives, otherwise the results were concordant. In one VZV-positive sample a very weak HHV-6B microarray signal was seen in addition to VZV signal (Table VII-).

The herpesvirus-negative panel (70 serum, 30 CSF and 11 proficiency-testing samples) was negative in multiplex-PCRs and microarray. Thus the specificity turned out to be 100% (results not included in the tables).

**TABLE VIII. Every-day diagnostic PCR and microarray results of QCMD specimens.**

| PCR result (number of specimens) | Microarray result (number of specimens) |
|---|---|
| neg (2) | neg (2) |
| HSV-1 (2) | HSV-1 (1) |
| | neg (1) |
| HSV-2 (2) | HSV-2 (1) |
| | neg (1) |
| VZV (4) | VZV (3) |
| | VZV and HHV-6B (1) |

| | |
|---|---|
| neg, negative | |

### REFERENCES

Aalto SM, Juvonen E, Tarkkanen J, Volin L, Ruutu T, Mattila PS, Piiparinen H, Knuutila S, Hedman K. Lymphoproliferative disease after allogeneic stem cell transplantation - pre-emptive diagnosis by quantification of Epstein-Barr virus DNA in serum. J. Clin. Virol. 2003;28:275-83.
Aberle SW, Puchhammer-Stockl E. Diagnosis of herpesvirus infections of the central nervous system. J. Clin. Virol. 2002;1:S79-85.
Akhtar et al. PCR diagnosis of viral pneumonitis from fixed-lung tissue in children. Biochemical and Molecular Medicine 1996; 58:66-76.
Aurelius E, Johansson B, Sköldenberg B, Staland A, Forsgren M. Rapid diagnosis of herpes simplex encephalitis by nested polymerase chain reaction assay of cerebrospinal fluid. Lancet 1991;337:189-92.
Boriskin YS, Rice PS, Stabler RA, Hinds J, Al-Ghusein H, Vass K, Butcher PD. DNA microarrays for virus detection in cases of central nervous system infection. J. Clin. Microbiol. 2004;42:5811-8.
Borsting C, Sanchez JJ, Morling N. Multiplex PCR, amplicon size and hybridization efficiency on the NanoChip electronic microarray. Int. J. Legal Med. 2004;118:75-82.
Boutolleau D, Fernandez C, Andre E, Imbert-Marcille BM, Milpied N, Agut H, Gautheret-Dejean A. Human herpesvirus (HHV)-6 and HHV-7: two closely related viruses with different infection profiles in stem cell transplantation recipients. J. Infect. Dis. 2003;187:179-86.
Chiu SS, Cheung CY, Tse CY, Peiris M. Early diagnosis of primary human herpesvirus 6 infection in childhood: serology, polymerase chain reaction, and virus load. J. Infect. Dis. 1998;178:1250-6.
Chizhikov V, Wagner M, Ivshina A, Hoshino Y, Kapikian AZ, Chumakov K. Detection and genotyping of human group A rotaviruses by oligonucleotide microarray hybridization. J. Clin. Microbiol. 2002;40:2398-407.
Druce J, Catton M, Chibo D, Minerds K, Tyssen D, Kostecki R, Maskill B, Leong-Shaw W, Gerrard M, Birch C. Utility of a multiplex PCR assay for detecting herpesvirus DNA in clinical samples. J. Clin. Microbiol. 2002;40:1728-32.
Dockrell DH, Paya CV. Human herpesvirus-6 and -7 in transplantation. Rev. Med. Virol. 2001;11:23-36.
Echevarria JM, Casas I, Tenorio A, de Ory F, Martinez-Martin P. Detection of varicella-zoster virus-specific DNA sequences in cerebrospinal fluid from patients with acute aseptic meningitis and no cutaneous lesions. J. Med. Virol. 1994;43:331-5.
Espy MJ, Teo R, Ross TK, Svien KA, Wold AD, Uhl JR, Smith TF. Diagnosis of varicella-zoster virus infections in the clinical laboratory by LightCycler PCR. J. Clin. Microbiol. 2000;38:3187-9.
Földes-Papp Z, Egerer R, Birch-Hirschfeld E, Striebel H-M, Demel U, Tolz G P and Wutzler P. Detection of multiple human herpes viruses by DNA microarray technology. Mol Diagn 2004; 8(1):1-9.
Gemignani F, Landi S, Chabrier A, Smet A, Zehbe I, Canzian F, Tommasino M. Generation of a DNA microarray for determination of E6 natural variants of human papillomavirus type 16. J. Virol. Methods 2004;119:95-102.
Gopal MR, Thomson BJ, Fox J, Tedder RS, Honess RW. Detection by PCR of HHV-6 and EBV DNA in blood and oropharynx of healthy adults and HIV-seropositives. Lancet 1990;335:1598-9.
Guo Z, Guilfoyle RA, Thiel AJ, Wang R, Smith LM. Direct fluorescence analysis of genetic polymorphisms by hybridization with oligonucleotide arrays on glass supports. Nucleic Acids Res. 1994;22:5456-65.
Hermouet S, Sutton CA, Rose TM, Greenblatt RJ, Corre I, Garand R, Neves AM, Bataille R, Casey JW. Qualitative and quantitative analysis of human herpesviruses in chronic and acute B cell lymphocytic leukemia and in multiple myeloma. Leukemia 2003;17:185-95.
Huber M, Mundlein A, Dornstauder E, Schneeberger C, Tempfer CB, Mueller MW, Schmidt WM. Accessing single nucleotide polymorphisms in genomic DNA by direct multiplex polymerase chain reaction amplification on oligonucleotide microarrays. Anal. Biochem. 2002;303:25-33.
Hudnall SD, Chen T, Tyring SK. Species identification of all eight human herpesviruses with a single nested PCR assay. J. Virol. Methods 2004;116:19-26.
Ihira M, Yoshikawa T, Ishii J, Nomura M, Hishida H, Ohashi M, Enomoto Y, Suga S, Iida K, Saito Y, Nishiyama Y, Asano Y. Serological examination of human herpesvirus 6 and 7 in patients with coronary artery disease. J. Med. Virol. 2002;67:534-7.
Kimura H, Morita M, Yabuta Y, Kuzushima K, Kato K, Kojima S, Matsuyama T, Morishima T. Quantitative analysis of Epstein-Barr virus load by using a real-time PCR assay. J. Clin. Microbiol. 1999;37:132-6.
Koskiniemi M, Mannonen L, Kallio A, Vaheri A. Luminometric microplate hybridization for detection of varicella-zoster virus PCR product from cerebrospinal fluid. J. Virol. Methods 1997;63:71-9.
Koskiniemi M, Piiparinen H, Rantalaiho T, Eränko P, Färkkilä M, Räihä K, Salonen EM, Ukkonen P, Vaheri A. Acute central nervous system complications in varicella zoster virus infections. J. Clin. Virol. 2002;25:293-301.
Lindroos K, Liljedahl U, Raitio M, Syvanen AC. Minisequencing on oligonucleotide microarrays: comparison of immobilisation chemistries. Nucleic Acids Res. 2001 Jul 1;29(13):E69-9.
Pastinen T, Raitio M, Lindroos K, Tainola P, Peltonen L, Syvänen AC. A system for specific, high-throughput genotyping by allele-specific primer extension on microarrays. Genome Res. 2000;10:1031-42.
Piiparinen H, Vaheri A. Genotyping of herpes simplex viruses by polymerase chain reaction. Arch. Virol. 1991;119:275-283.
Piiparinen H, Höckerstedt K, Lappalainen M, Suni J, Lautenschlager I. Monitoring of viral load by quantitative plasma PCR during active cytomegalovirus infection of individual liver transplant patients. J. Clin. Microbiol. 2002;40:2945-52.
Piiparinen H, Höckerstedt K, Grönhagen-Riska C, Lautenschlager I. Comparison of two quantitative CMV PCR tests, Cobas Amplicor CMV Monitor and TaqMan assay, and pp65-antigenemia assay in the determination of viral loads from peripheral blood of organ transplant patients. J. Clin. Virol. 2004;30:258-66.
Pitkäranta A, Piiparinen H, Mannonen L, Vesaluoma M, Vaheri A. Detection of human herpesvirus 6 and varicella-zoster virus in tear fluid of patients with Bell's palsy by PCR. J. Clin. Microbiol. 2000;38:2753-5.
Read SJ, Kurtz JB. Laboratory diagnosis of common viral infections of the central nervous system by using a single multiplex PCR screening assay. J. Clin. Microbiol. 1999;37:1352-5.
Read SJ, Mitchell JL, Fink CG. LightCycler multiplex PCR for the laboratory diagnosis of common viral infections of the central nervous system. J. Clin. Microbiol. 2001;39:3056-9.
Rozen S, Skaletsky H. Primer3 on the WWW for general users and for biologist programmers. Methods Mol. Biol. 2000;132:365-86.
Safronetz D, Humar, A, Tipples, G A. Differentation and quantitation of human herpesviruses 6A, 6B, and by real-time PCR. Journal of Virological Methods 2003, 112:99-105.
Schirm J, J. J. Meulenberg JJ, Pastoor GW, van Voorst Vader PC, Schroder FP. Rapid detection of varicella-zoster virus in clinical specimens using monoclonal antibodies on shell vials and smears. J. Med. Virol. 1989;28:1-6.
Sengupta S, Onodera K, Lai A, Melcher U. Molecular detection and identification of influenza viruses by oligonucleotide microarray hybridization. J. Clin. Microbiol. 2003;41:4542-50.
Striebel H-M, Birch-Hirschfeld E, Egerer R, Földes-Papp Z, Tilz G P, Stelzner A. Enhancing sensitivity of human herpes virus diagnosis with DANN microarrays using dendrimers 2004;77:89-97
Suga S, Yoshikawa T, Nagai T, Asano Y. Clinical features and virological findings in children with primary human herpesvirus 7 infection. Pediatrics 1997;99:E4.
Tanaka K, Kondo T, Torigoe S, Okada S, Mukai T, Yamanishi K. 1994. Human herpesvirus 7: another causal agent for roseola (exanthem subitum). J. Pediatr. 1994;125:1-5.
Van den Veyver et al. Detection of intrauterine viral infection using the polymerase chain reaction. Molecular Genetics and Metabolism 1998; 63:85-95.
Vesanen M, Piiparinen H, Kallio A, Vaheri A. Detection of herpes simplex virus DNA in cerebrospinal fluid samples using the polymerase chain reaction and microplate hybridization. J. Virol. Methods 1996;59:1-11.
Volokhov D, Chizhikov V, Chumakov K, Rasooly A. Microarray-based identification of thermophilic Campylobacter jejuni, C. coli, C. lari, and C. upsaliensis. J. Clin. Microbiol. 2003;41:4071-80.
Wang D, Coscoy L, Zylberberg M, Avila PC, Boushey HA, Ganem D, DeRisi JL. Microarray-based detection and genotyping of viral pathogens. Proc. Natl. Acad. Sci. USA 2002;99:15687-92.
Zuker M. Mfold web server for nucleic acid folding and hybridization prediction. Nucleic Acids Res. 2003;13:3406-3415.
Yamamoto T, Nakamura, Y. A single tube PCR assay for simultaneous amplification of HSV-1/-2, VZV, CMV, HHV-6A/-6B, and EBV DNAs in cerebrospinal fluid from patients with virus-related neurological diseases. Journal of NeuroVirology 2000; 6:410-417.

## Claims

1. A method for detecting one or more herpesviruses in a biological sample, which comprises the steps:
- extracting DNA from the biological sample;
- amplifying the extracted DNA;
- translating the amplified DNA to ssRNA;
- hybridizing ssRNAs to oligonucleotide sequences on a microarray, said oligonucleotide sequences corresponding to each of the herpesviruses to be detected;
- extending the hybridized ssRNA by primer extension method in the presence of detectable nucleotides;
- detecting a signal from the detectable nucleotides by a suitable method;
wherein the length of the oligonucleotide sequence is less than 50 nucleotides and the sequence comprises a sequence in 5' to 3' direction selected from the group comprising SEQ ID NO: 4, SEQ ID NO: 5, SEQ ID NO: 8, SEQ ID NO: 11, SEQ ID NO: 14, SEQ ID NO: 17, SEQ ID NO: 18, SEQ ID NO: 21, SEQ ID NO: 24, SEQ ID NO: 25, SEQ ID NO: 26, SEQ ID NO: 27, SEQ ID NO: 28 and SEQ ID NO: 29, or a sequence lacking one nucleotide, having one additional nucleotide, or having one change in the nucleotide sequence compared to the sequences SEQ ID NO: 4, SEQ ID NO: 5, SEQ ID NO: 8, SEQ ID NO: 11, SEQ ID NO: 14, SEQ ID NO: 17, SEQ ID NO: 18, SEQ ID NO: 21, SEQ ID NO: 24, SEQ ID NO: 25, SEQ ID NO: 26, SEQ ID NO: 27, SEQ ID NO: 28 or SEQ ID NO: 29.

2. The method according to claim 1, wherein the microarray comprises oligonucleotides corresponding to at least two different herpesviruses, preferably at least three different herpesviruses.

3. The method according to claim 1 or 2, wherein the microarray comprises oligonucleotides corresponding to herpesviruses selected from the group comprising
HSV-1, HSV-2, CMV, EBV, VZV, HHV-6A, HHV-6B and HHV-7.

4. The method according to any one of claims 1 to 3, wherein the detection of different herpesviruses is simultaneous.

5. The method according to any one of the preceding claims, wherein the method further comprises the detection of other pathogens, preferably viruses.

6. The method according to any one of the preceding claims, wherein the method further comprises the detection of diseases of the central nervous system (CNS) or immunodeficiency diseases.

7. The method according to any one of the preceding claims, wherein the method further comprises the detection of enteroviruses of borrelia.

8. The method according to any one of the preceding claims, wherein the detection of different herpesviruses and other pathogens or diseases is simultaneous.

9. The method according to any one of the preceding claims, wherein the amplification of the DNA is carried out by using a pair of primers comprising primer A and primer B selected from the group comprising
A is SEQ ID NO: 22 and B is SEQ ID NO: 23,
A is SEQ ID NO: 6 and B is SEQ ID NO: 7;
A is SEQ ID NO: 9 and B is SEQ ID NO: 10;
A is SEQ ID NO: 15 and B is SEQ ID NO: 16, and
A is SEQ ID NO: 19 and B is SEQ ID NO: 20,
or a sequence having at least 90 % identity with the mentioned sequences.

10. The method according to any one of the preceding claims, wherein the amplification of the DNA is carried out by multiplex PCR in two reactions.

11. The method according to claim 10, wherein HSV-1 and HSV-2 are amplified in one reaction and CMV, EBV, VZV, HHV-6A, HHV-6B and HHV-7 are amplified in another reaction.

12. A microarray for detecting herpesviruses, which comprises
- a solid support comprising at least one array;
- said array comprising oligonucleotides specific for at least one herpesvirus, wherein the length of the oligonucleotide sequence is less than 50 nucleotides and the sequence comprises a sequence in 5' to 3' direction selected from the group consisting of SEQ ID NO: 5, SEQ ID NO: 25, SEQ ID NO: 26 and SEQ ID NO: 27, or a sequence lacking one nucleotide, having one additional nucleotide, or having one change in the nucleotide sequence compared to the sequences SEQ ID NO: 5, SEQ ID NO: 25, SEQ ID NO: 26 or SEQ ID NO: 27.

13. The microarray according to claim 12, wherein the number of arrays on the solid support is 1 to 80, preferably 10 to 60.

14. The microarray according to claim 12 or 13, wherein the number of oligonucleotide spots on a microarray is 4 to 200, preferably 10 to 100.

15. The microarray according to any one of claim 12 to 14, wherein the microarray is used for detecting a herpesvirus selected from the group comprising
HSV-1, HSV-2, CMV, EBV, VZV, HHV-6A, HHV-6B and HHV-7.

16. The microarray according to any one of claim 12 to 15, wherein the microarray comprises oligonucleotides for detecting further diseases as those caused by herpesvirus, such as diseases of the central nervous system (CNS) or immunodeficiency diseases, or oligonucleotides for detecting other pathogens, preferably viruses.

17. The microarray according to any one of claim 12 to 16, wherein the microarray comprises oligonucleotides for detecting other pathogens, such as enteroviruses or borrelia causing pathogens.

18. A kit which comprises the microarray according to any one of claims 12 to 17 and optionally, primers, buffers, enzymes and/or nucleotides.

19. A method for preparing a microarray for detecting herpesviruses, which comprises
- attaching oligonucleotides corresponding to at least one herpesvirus in an array;
- multiplying the arrays onto a slide, the number of the arrays corresponding to the number of samples to be analysed.
wherein the length of the oligonucleotide sequence is less than 50 nucleotides and the sequence comprises a sequence in 5' to 3' direction selected from the group consisting of SEQ ID NO: 5, SEQ ID NO: 25, SEQ ID NO: 26 and SEQ ID NO: 27, or a sequence lacking one nucleotide, having one additional nucleotide, or having one change in the nucleotide sequence compared to the sequences SEQ ID NO: 5, SEQ ID NO: 25, SEQ ID NO: 26 or SEQ ID NO: 27.

20. An oligonucleotide comprising a sequence selected from the group consisting of:
SEQ ID NO: 5, SEQ ID NO: 25, SEQ ID NO: 26, and SEQ ID NO: 27, wherein the length of the oligonucleotide sequence is less than 50 nucleotides and the sequence comprises the mentioned sequence in 5' to 3' direction.

21. A primer pair consisting of a primer A comprising SEQ ID NO: 22 and a primer B comprising SEQ ID NO: 23 or a sequence having at least 95 % identity with the mentioned sequences.

22. A set of multiplex primer pairs comprising a primer pair according to claim 21 and one or more further primer pairs selected from the group consisting of
a primer pair of a primer A comprising SEQ ID NO: 6 and a primer B comprising SEQ ID NO:7,
a primer pair of a primer A comprising SEQ ID NO: 9 and a primer B comprising SEQ ID NO:10,
a primer pair of a primer A comprising SEQ ID NO: 12 and a primer B comprising SEQ ID NO:13,
a primer pair of a primer A comprising SEQ ID NO: 15 and a primer B comprising SEQ ID NO:16, and
a primer pair of a primer A comprising SEQ ID NO: 19 and a primer B comprising SEQ ID NO:20,
said primer pairs comprising sequences having at least 95 % identity with the mentioned sequences.

23. A set of multiplex primer pairs comprising all primer pairs set forth in claim 22.

24. A method for detecting at least two herpes viruses simultaneously in a biological sample, which comprises the steps:
- extracting DNA from a biological sample;
- amplifying the extracted DNA;
- translating the amplified DNA to ssRNA;
- hybridizing ssRNAs to oligonucleotide sequences on a microarray plate, said oligonucleotide sequences corresponding to herpesviruses commonly causing simultaneous infections;
- extending the hybridized ssRNA by primer extension method in the presence of detectable nucleotides; and
- detecting a signal from the detectable nucleotides by a suitable method.

## Patentansprüche

1. Verfahren zum Nachweisen von einem oder mehreren Herpesviren in einer biologischen Probe, welches die Schritte umfasst:
- Extrahieren von DNA aus der biologischen Probe;
- Amplifizieren der extrahierten DNA;
- Translatieren der amplifizierten DNA zu ssRNA;
- Hybridisieren von ssRNAs mit Oligonukleotid-Sequenzen auf einem Mikroarray, wobei die Oligonukleotid-Sequenzen jedem der nachzuweisenden Herpesviren entsprechen;
- Verlängern der hybridisierten ssRNA durch Primer-Extensions-Verfahren in Gegenwart von nachweisbaren Nukleotiden;
- Nachweisen eines Signals aus den nachweisbaren Nukleotiden durch ein geeignetes Verfahren;
wobei die Länge der Oligonukleotid-Sequenz weniger als 50 Nukleotide ist und die Sequenz eine Sequenz in 5' nach 3' Richtung, ausgewählt aus der Gruppe, umfassend SEQ ID NR.: 4, SEQ ID NR.: 5, SEQ ID NR.: 8, SEQ ID NR.: 11, SEQ ID NR.: 14, SEQ ID NR.: 17, SEQ ID NR.: 18, SEQ ID NR.:21, SEQ ID NR.: 24, SEQ ID NR.: 25, SEQ ID NR.: 26, SEQ ID NR.: 27, SEQ ID NR.: 28 und SEQ ID NR.: 29, oder eine Sequenz, der ein Nukleotid fehlt, die ein zusätzliches Nukleotid aufweist, oder eine Änderung in der Nukleotid-Sequenz aufweist, verglichen mit den Sequenzen SEQ ID NR.: 4, SEQ ID NR.: 5, SEQ ID NR.: 8, SEQ ID NR.: 11, SEQ ID NR.: 14, SEQ ID NR.: 17, SEQ ID NR.: 18, SEQ ID NR.: 21, SEQ ID NR.: 24, SEQ ID NR.: 25, SEQ ID NR.: 26, SEQ ID NR.: 27, SEQ ID NR.: 28 oder SEQ ID NR.: 29, umfasst.

2. Verfahren nach Anspruch 1, wobei das Mikroarray Oligonukleotide, entsprechend mindestens zwei verschiedenen Herpesviren, vorzugsweise mindestens drei verschiedenen Herpesviren, umfasst.

3. Verfahren nach Anspruch 1 oder 2, wobei das Mikroarray Oligonukleotide, entsprechend Herpesviren, ausgewählt aus der Gruppe, umfassend HSV-1, HSV-2, CMV, EBV, VZV, HHV-6A, HHV-6B und HHV-7, umfasst.

4. Verfahren nach einem der Ansprüche 1 bis 3, wobei der Nachweis der verschiedenen Herpesviren gleichzeitig erfolgt.

5. Verfahren nach einem der vorangehenden Ansprüche, wobei das Verfahren weiterhin den Nachweis von anderen Pathogenen, vorzugsweise Viren, umfasst.

6. Verfahren nach einem der vorangehenden Ansprüche, wobei das Verfahren weiterhin den Nachweis von Krankheiten des zentralen Nervensystems (ZNS) oder Immun-Mangel-Krankheiten umfasst.

7. Verfahren nach einem der vorangehenden Ansprüche, wobei das Verfahren weiterhin den Nachweis von Enteroviren von Borrelien umfasst.

8. Verfahren nach einem der vorangehenden Ansprüche, wobei der Nachweis von verschiedenen Herpesviren und anderen Pathogenen oder Krankheiten gleichzeitig erfolgt.

9. Verfahren nach einem der vorangehenden Ansprüche, wobei die Amplifikation der DNA durch Anwenden eines Paars von Primern, umfassend Primer A und Primer B, ausgewählt aus der Gruppe, umfassend
A ist SEQ ID NR.: 22 und B ist SEQ ID NR.: 23,
A ist SEQ ID NR.: 6 und B ist SEQ ID NR.: 7;
A ist SEQ ID NR.: 9 und B ist SEQ ID NR.: 10;
A ist SEQ ID NR.: 15 und B ist SEQ ID NR.: 16, und
A ist SEQ ID NR.: 19 und B ist SEQ ID NR.: 20, oder eine Sequenz, die mindestens 90 % Identität mit den erwähnten Sequenzen aufweist, ausgeführt wird.

10. Verfahren nach einem der vorangehenden Ansprüche, wobei die Amplifikation der DNA durch Multiplex PCR in zwei Reaktionen ausgeführt wird.

11. Verfahren nach Anspruch 10, wobei HSV-1 und HSV-2 in einer Reaktion amplifiziert werden und CMV, EBV, VZV, HHV-6A, HHV-6B und HHV-7 in einer weiteren Reaktion amplifiziert werden.

12. Mikroarray zum Nachweisen von Herpesviren, welches umfasst
- einen festen Träger, umfassend mindestens ein Array;
- wobei das Array Oligonukleotide, die für mindestens einen Herpesvirus spezifisch sind, umfasst, wobei die Länge der Oligonukleotid-Sequenz weniger als 50 Nukleotide ist und die Sequenz eine Sequenz in 5' nach 3' Richtung, ausgewählt aus der Gruppe, bestehend aus SEQ ID NR.: 5, SEQ ID NR.: 25, SEQ ID NR.: 26 und SEQ ID NR.: 27 oder eine Sequenz, der ein Nukleotid fehlt, die ein zusätzliches Nukleotid aufweist, oder eine Änderung in der Nukleotid-Sequenz aufweist, verglichen mit den Sequenzen SEQ ID NR.: 5, SEQ ID NR.: 25, SEQ ID NR.: 26 oder SEQ ID NR.: 27, umfasst.

13. Mikroarray nach Anspruch 12, wobei die Anzahl der Arrays auf dem festen Träger 1 bis 80, vorzugsweise 10 bis 60, ist.

14. Mikroarray nach Anspruch 12 oder 13, wobei die Anzahl von Oligonukleotid-Flecken auf einem Mikroarray 4 bis 200, vorzugsweise 10 bis 100, ist.

15. Mikroarray nach einem von Anspruch 12 bis 14, wobei das Mikroarray zum Nachweisen eines Herpesvirus, ausgewählt aus der Gruppe, umfassend HSV-1, HSV-2, CMV, EBV, VZV, HHV-6A, HHV-6B und HHV-7, verwendet wird.

16. Mikroarray nach einem von Anspruch 12 bis 15, wobei das Mikroarray Oligonukleotide zum Nachweisen weiterer Krankheiten wie jene, verursacht durch Herpesvirus, wie Krankheiten des zentralen Nervensystems (ZNS) oder Immun-Mangel-Krankheiten, oder Oligonukleotide zum Nachweisen anderer Pathogene, vorzugsweise Viren, umfasst.

17. Mikroarray nach einem von Anspruch 12 bis 16, wobei das Mikroarray Oligonukleotide zum Nachweisen anderer Pathogene, wie Enteroviren oder Borrelien verursachende Pathogene, umfasst.

18. Kit, welches das Mikroarray nach einem von Ansprüchen 12 bis 17 und gegebenenfalls Primer, Puffer, Enzyme und/oder Nukleotide umfasst.

19. Verfahren zum Herstellen eines Mikroarrays zum Nachweisen von Herpesviren, welches umfasst
- Anbringen von Oligonukleotiden, die mindestens einem Herpesvirus entsprechen, in einem Array;
- Vervielfältigen der Arrays auf einem Objektträger, wobei die Anzahl der Arrays der Anzahl von zu analysierenden Proben entspricht,
wobei die Länge der Oligonukleotid-Sequenz weniger als 50 Nukleotide ist und die Sequenz eine Sequenz in 5' nach 3' Richtung, ausgewählt aus der Gruppe, bestehend aus SEQ ID NR.: 5, SEQ ID NR.: 25, SEQ ID NR.: 26 und SEQ ID NR.: 27 oder eine Sequenz, der ein Nukleotid fehlt, die ein zusätzliches Nukleotid aufweist, oder eine Änderung in der Nukleotid-Sequenz aufweist, verglichen mit den Sequenzen SEQ ID NR.: 5, SEQ ID NR.: 25, SEQ ID NR.: 26 oder SEQ ID NR.: 27, umfasst.

20. Oligonukleotid, umfassend eine Sequenz, ausgewählt aus der Gruppe, bestehend aus:
SEQ ID NR.: 5, SEQ ID NR.: 25, SEQ ID NR.: 26 und SEQ ID NR.: 27, wobei die Länge der Oligonukleotid-Sequenz weniger als 50 Nukleotide ist und die Sequenz die erwähnte Sequenz in 5' nach 3' Richtung umfasst.

21. Primerpaar, bestehend aus einem Primer A, umfassend SEQ ID NR.: 22, und einem Primer B, umfassend SEQ ID NR.: 23, oder eine Sequenz mit mindestens 95 % Identität mit den erwähnten Sequenzen.

22. Reihe von Multiplex-Primerpaaren, umfassend ein Primerpaar nach Anspruch 21 und eines oder mehrere weitere Primerpaare, ausgewählt aus der Gruppe, bestehend aus einem Primerpaar von einem Primer A, umfassend SEQ ID NR.: 6, und einem Primer B, umfassend SEQ ID NR.:7,
einem Primerpaar von einem Primer A, umfassend SEQ ID NR.: 9, und einem Primer B, umfassend SEQ ID NR.:10,
einem Primerpaar von einem Primer A, umfassend SEQ ID NR.: 12, und einem Primer B, umfassend SEQ ID NR.:13,
einem Primerpaar von einem Primer A, umfassend SEQ ID NR.: 15, und einem Primer B, umfassend SEQ ID NR.:16, und
einem Primerpaar von einem Primer A, umfassend SEQ ID NR.: 19, und einem Primer B, umfassend SEQ ID NR.:20,
wobei die Primerpaare Sequenzen mit mindestens 95 % Identität mit den erwähnten Sequenzen umfassen.

23. Reihe von Multiplex-Primerpaaren, umfassend alle Primerpaare wie in Anspruch 22 angeführt.

24. Verfahren zum Nachweisen von mindestens zwei Herpes-Viren gleichzeitig in einer biologischen Probe, welches die Schritte umfasst:
- Extrahieren von DNA aus einer biologischen Probe;
- Amplifizieren der extrahierten DNA;
- Translatieren der amplifizierten DNA zu ssRNA;
- Hybridisieren von ssRNAs zu Oligonukleotid-Sequenzen auf einer Mikroarrayplatte, wobei die Oligonukleotid-Sequenzen den Herpesviren, die üblicherweise gleichzeitig Infektionen verursachen, entsprechen;
- Verlängern der hybridisierten ssRNA durch Primer-Extensions-Verfahren in Gegenwart von nachweisbaren Nukleotiden; und
- Nachweisen eines Signals aus den nachweisbaren Nukleotiden durch ein geeignetes Verfahren.

## Revendications

1. Procédé de détection d'un ou plusieurs virus de l'herpès dans un échantillon biologique, qui comprend les étapes consistant à :
- extraire de l'ADN de l'échantillon biologique ;
- amplifier l'ADN extrait ;
- traduire l'ADN amplifié en ARNss ;
- hybrider les ARNss avec des séquences oligonucléotidiques sur un microréseau, lesdites séquences oligonucléotidiques correspondant à chacun des virus de l'herpès à détecter ;
- étendre l'ARNss hybridé par un procédé d'extension d'amorces en présence de nucléotides détectables ;
- détecter un signal issu des nucléotides détectables par un procédé approprié ; dans lequel la longueur de la séquence oligonucléotidique est inférieure à 50 nucléotides et la séquence comprend une séquence dans le sens 5' à 3' choisie dans le groupe comprenant les SEQ ID NO : 4, SEQ ID NO : 5, SEQ ID NO : 8, SEQ ID NO : 11, SEQ ID NO : 14, SEQ ID NO : 17, SEQ ID NO : 18, SEQ ID NO : 21, SEQ ID NO : 24, SEQ ID NO : 25, SEQ ID NO : 26, SEQ ID NO : 27, SEQ ID NO : 28 et SEQ ID NO : 29, ou une séquence manquant d'un nucléotide, ayant un nucléotide supplémentaire, ou ayant un changement dans la séquence nucléotidique en comparaison des séquences SEQ ID NO : 4, SEQ ID NO : 5, SEQ ID NO : 8, SEQ ID NO : 11, SEQ ID NO : 14, SEQ ID NO : 17, SEQ ID NO : 18, SEQ ID NO : 21, SEQ ID NO : 24, SEQ ID NO : 25, SEQ ID NO : 26, SEQ ID NO : 27, SEQ ID NO : 28 et SEQ ID NO : 29.

2. Procédé selon la revendication 1, dans lequel le microréseau comprend des oligonucléotides correspondant à au moins deux virus différents de l'herpès, de préférence au moins trois virus différents de l'herpès.

3. Procédé selon la revendication 1 ou 2, dans lequel le microréseau comprend des oligonucléotides correspondant à des virus de l'herpès choisis dans le groupe comprenant les suivants :
HSV-1, HSV-2, CMV, EBV, VZV, HHV-6A, HHV-6B et HHV-7.

4. Procédé selon l'une quelconque des revendications 1 à 3, dans lequel la détection de différents virus de l'herpès est simultanée.

5. Procédé selon l'une quelconque des revendications précédentes, dans lequel le procédé comprend en outre la détection d'autres agents pathogènes, de préférence des virus.

6. Procédé selon l'une quelconque des revendications précédentes, dans lequel le procédé comprend en outre la détection de maladies du système nerveux central (SNC) ou de maladies d'immunodéficience.

7. Procédé selon l'une quelconque des revendications précédentes, dans lequel le procédé comprend en outre la détection d'entérovirus de Borrelia.

8. Procédé selon l'une quelconque des revendications précédentes, dans lequel la détection de différents virus de l'herpès et d'autres agents pathogènes ou maladies est simultanée.

9. Procédé selon l'une quelconque des revendications précédentes, dans lequel l'amplification de l'ADN est effectuée en utilisant une paire d'amorces comprenant l'amorce A et l'amorce B sélectionnées dans le groupe comprenant les suivantes :
A est la SEQ ID NO : 22 et B est la SEQ ID NO : 23,
A est la SEQ ID NO : 6 et B est la SEQ ID NO : 7 ;
A est la SEQ ID NO : 9 et B est la SEQ ID NO : 10 ;
A est la SEQ ID NO : 15 et B est la SEQ ID NO : 16 et
A est la SEQ ID NO : 19 et B est la SEQ ID NO : 20,
ou une séquence ayant au moins 90 % d'identité avec les séquences mentionnées.

10. Procédé selon l'une quelconque des revendications précédentes, dans lequel l'amplification de l'ADN est effectuée par une PCR multiplex en deux réactions.

11. Procédé selon la revendication 10, dans lequel HSV-1 et HSV-2 sont amplifiés dans une réaction et CMV, EBV, VZV, HHV-6A, HHV-6B et HHV-7 sont amplifiés dans une autre réaction.

12. Microréseau pour détecter des virus de l'herpès, qui comprend :
- un support solide comprenant au moins un réseau ;
- ledit réseau comprenant des oligonucléotides spécifiques pour au moins un virus de l'herpès, dans lequel la longueur de la séquence oligonucléotidique est inférieure à 50 nucléotides et la séquence comprend une séquence dans le sens 5' à 3' sélectionnée dans le groupe constitué des SEQ ID NO : 5, SEQ ID NO : 25, SEQ ID NO : 26 et SEQ ID NO : 27,
ou une séquence manquant d'un nucléotide, ayant un nucléotide supplémentaire ou ayant un changement dans la séquence nucléotidique en comparaison des séquences SEQ ID NO : 5, SEQ ID NO : 25, SEQ ID NO : 26 ou SEQ ID NO : 27.

13. Microréseau selon la revendication 12, dans lequel le nombre de réseaux sur le support solide est de 1 à 80, de préférence de 10 à 60.

14. Microréseau selon la revendication 12 ou 13, dans lequel le nombre de tâches oligonucléotidiques sur un microréseau est de 4 à 200, de préférence de 10 à 100.

15. Microréseau selon l'une quelconque des revendications 12 à 14, dans lequel le microréseau est utilisé pour détecter un virus de l'herpès choisi dans le groupe comprenant les suivants :
HSV-1, HVS-2, CMV, EBV, VZV, HHV-6A, HHV-6B et HHV-7.

16. Microréseau selon l'une quelconque des revendications 12 à 15, dans lequel le microréseau comprend des oligonucléotides pour détecter d'autres maladies que celles provoquées par un virus de l'herpès, notamment des maladies du système nerveux central (SNC) ou des maladies d'immunodéficience, ou des oligonucléotides pour détecter d'autres agents pathogènes, de préférence des virus.

17. Microréseau selon l'une quelconque des revendications 12 à 16, dans lequel le microréseau comprend des oligonucléotides pour détecter d'autres agents pathogènes, tels que des entérovirus ou des agents pathogènes provoquant la Borrelia.

18. Kit comprenant le microréseau selon l'une quelconque des revendications 12 à 17 et éventuellement des amorces, des tampons, des enzymes et/ou des nucléotides.

19. Procédé de préparation d'un microréseau pour détecter des virus de l'herpès, qui comprend :
- la fixation d'oligonucléotides correspondant à au moins un virus de l'herpès dans un réseau ;
- la multiplication des réseaux sur une lame, le nombre des réseaux correspondant au nombre d'échantillons à analyser,
- dans lequel la longueur de la séquence oligonucléotidique est inférieure à 50 nucléotides et la séquence comprend une séquence dans le sens 5' à 3' sélectionnée dans le groupe constitué des SEQ ID NO : 5, SEQ ID NO : 25, SEQ ID NO : 26 et SEQ ID NO : 27,
ou une séquence manquant d'un nucléotide, ayant un nucléotide supplémentaire ou ayant un changement dans la séquence nucléotidique en comparaison des séquences SEQ ID NO : 5, SEQ ID NO : 25, SEQ ID NO : 26 ou SEQ ID NO : 27.

20. Oligonucléotide comprenant une séquence sélectionnée dans le groupe constitué des suivantes :
SEQ ID NO : 5, SEQ ID NO : 25, SEQ ID NO: 26 et SEQ ID NO: 27,
dans lequel la longueur de la séquence oligonucléotidique est inférieure à 50 nucléotides et la séquence comprend la séquence mentionnée dans le sens 5' à 3'.

21. Paire d'amorces constituée d'une amorce A comprenant la SEQ ID NO : 22 et d'une amorce B comprenant la SEQ ID NO : 23 ou d'une séquence ayant au moins 95 % d'identité avec les séquences mentionnées.

22. Jeu de paires d'amorces multiplex comprenant une paire d'amorces selon la revendication 21 et une ou plusieurs autres paires d'amorces choisies dans le groupe constitué des suivantes :
une paire d'amorces formée d'une amorce A comprenant la SEQ ID NO : 6 et d'une amorce B comprenant la SEQ ID NO : 7,
une paire d'amorces formée d'une amorce A comprenant la SEQ ID NO : 9 et d'une amorce B comprenant la SEQ ID NO : 10,
une paire d'amorces formée d'une amorce A comprenant la SEQ ID NO : 12 et d'une amorce B comprenant la SEQ ID NO : 13,
une paire d'amorces formée d'une amorce A comprenant la SEQ ID NO : 15 et d'une amorce B comprenant la SEQ ID NO : 16, et
une paire d'amorces formée d'une amorce A comprenant la SEQ ID NO : 19 et d'une amorce B comprenant la SEQ ID NO : 20,
lesdites paires d'amorces comprenant des séquences ayant au moins 95 % d'identité avec les séquences mentionnées.

23. Jeu de paires d'amorces multiplex comprenant toutes des paires d'amorces mentionnées dans la revendication 22.

24. Procédé de détection d'au moins deux virus de l'herpès simultanément dans un échantillon biologique, qui comprend les étapes consistant à :
- extraire de l'ADN de l'échantillon biologique ;
- amplifier l'ADN extrait ;
- traduire l'ADN amplifié en ARNss ;
- hybrider les ARNss avec des séquences oligonucléotidiques sur une lame de microréseau, lesdites séquences oligonucléotidiques correspondant à des virus de l'herpès provoquant communément des infections simultanées ;
- étendre le ARNss hybridé par un procédé d'extension d'amorces en présence de nucléotides détectables ; et
- détecter un signal issu des nucléotides détectables par un procédé approprié.
